# EUROPEAN PATENT SPECIFICATION

(11) **EP 3 870 027 B1**
(45) Date of publication and mention of the grant of the patent: **09.10.2024**
(21) Application number: 18796869.8
(22) Date of filing: 25.10.2018
(51) Int. Cl.: A61B 1/00, A61B 1/04, A61B 1/31, A61B 5/00, G01N 33/574

(54) **PROCESS FOR VISUAL DETERMINATION OF TISSUE BIOLOGY**
VERFAHREN ZUR VISUELLEN BESTIMMUNG DES GEWEBEBIOLOGIE
PROCÉDÉ DE DÉTERMINATION VISUELLE DE LA BIOLOGIE TISSULAIRE

(43) Date of publication of application: 01.09.2021
(73) Proprietor: University College Dublin, National University of Ireland, Dublin, Dublin 4 (IE); The Royal College of Surgeons in Ireland, Dublin 2 (IE)
(72) Inventor: CAHILL, Ronan, Belfield, Dublin 4 (IE); O'SHEA, Donal, F., Dublin 2 (IE)
(74) Representative: Appleyard Lees IP LLP
(86) International application number: PCT/EP2018/079359
(87) International publication number: WO 2020/083501

(56) References cited:
- WO-A1-2015/198065
- US-A1- 2015 018 690
- US-A1- 2015 216 398
- A. POELLINGER ET AL: "Breast Cancer: Early- and Late-Fluorescence Near-Infrared Imaging with Indocyanine Green--A Preliminary Study", RADIOLOGY, vol. 258, no. 2, 1 February 2011 (2011-02-01), pages 409 - 416, XP055042425, ISSN: 0033-8419, DOI: 10.1148/radiol.10100258
- FLORIAN SCHMIDT ET AL: "Feasibility of real-time near-infrared indocyanine green fluorescence endoscopy for the evaluation of mucosal head and neck lesions : Near-Infrared Endoscopy for Head and Neck Lesions", HEAD AND NECK., vol. 39, no. 2, 2 September 2016 (2016-09-02), US, pages 234 - 240, XP055557478, ISSN: 1043-3074, DOI: 10.1002/hed.24570
- ANAND T. N. KUMAR ET AL: "Fluorescence lifetime-based contrast enhancement of indocyanine green-labeled tumors", INTERNATIONAL SOCIETY FOR OPTICAL ENGINEERING, vol. 22, no. 4, 11 April 2017 (2017-04-11), PO Box 10 Bellingham WA 98227-0010 USA, pages 040501, XP055557481, ISSN: 1083-3668, DOI: 10.1117/1.JBO.22.4.040501
- NOBUHIKO ONDA ET AL: "Preferential tumor cellular uptake and retention of indocyanine green for in vivo tumor imaging : In Vivo Tumor Imaging Using Indocyanine Green", INTERNATIONAL JOURNAL OF CANCER, vol. 139, no. 3, 1 August 2016 (2016-08-01), US, pages 673 - 682, XP055557487, ISSN: 0020-7136, DOI: 10.1002/ijc.30102
- REI NAGAHARA ET AL: "Fluorescence tumor imaging by i.v. administered indocyanine green in a mouse model of colitis-associated colon cancer", CANCER SCIENCE, vol. 109, no. 5, 19 April 2018 (2018-04-19), JP, pages 1638 - 1647, XP055557490, ISSN: 1347-9032, DOI: 10.1111/cas.13564
- JACK X JIANG ET AL: "Original Article Optimization of the enhanced permeability and retention effect for near-infrared imaging of solid tumors with indocyanine green", AM J NUCL MED MOL IMAGING, 1 January 2015 (2015-01-01), XP055557496
- NOBUHIKO ONDA ET AL: "Fluorescence contrast-enhanced proliferative lesion imaging by enema administration of indocyanine green in a rat model of colon carcinogenesis", ONCOTARGET, vol. 8, no. 52, 27 October 2017 (2017-10-27), XP055557501, DOI: 10.18632/oncotarget.21744
- WIBOOL PIYAWATTANAMETHA ET AL: "In vivo near-infrared dual-axis confocal microendoscopy in the human lower gastrointestinal tract", INTERNATIONAL SOCIETY FOR OPTICAL ENGINEERING, vol. 17, no. 2, 1 January 2012 (2012-01-01), PO Box 10 Bellingham WA 98227-0010 USA, pages 021102, XP055557506, ISSN: 1083-3668, DOI: 10.1117/1.JBO.17.2.021102

## Description

### Field

The present invention relates to a method of analysing a target bodily tissue and a device adapted to carry out said method. In particular the invention relates to a method of analysing lesions in a subject to assist with determining whether said lesions are cancerous.

### Background

Colorectal cancer is the development of cancerous growths in the colon or rectum. Patients with colorectal cancer may present with symptoms such as blood in the stool, a change in bowel movements, weight loss and fatigue. The development of colorectal cancer usually starts with a benign tumour in the form of a polyp, which becomes cancerous over time. The treatment options for colorectal cancer include excision of the lesion or some combination of surgery, radiation therapy and chemotherapy. The likelihood of a patient surviving colorectal cancer may be highly dependent on how early the cancer is detected and when treatment begins.

Lesions in the gastrointestinal tract, such as rectal lesions which may lead to colorectal cancer, are initially investigated by endoscopy. Such lesions are sometimes benign and require no clinical intervention. It has generally not been possible to make a positive determination of the benign or malignant state of such lesions by such observations alone. Therefore biopsies of such lesions are commonly taken and subjected to histological analysis to assist in the diagnosis of the lesion. However, this analysis of biopsied tissue may give falsely negative or ambiguous results which may result in over or under treatment of the patient and therefore lead to less than optimal outcomes for patients. The biopsies themselves can cause adverse effects in the patient. Similarly, when dealing with more advanced cancers, there may be deposits of disease at sites other then the primary site and these too need identification and possible intervention dependent on their correct classification as cancerous.

Imaging methods using contrast agents have been used in some situations to analyse suspect tissue formations in patients. For example narrowed spectrum endoscopy (such as narrowband imaging [NBI], image-enhanced endoscopy [i-scan], and Fujinon intelligent chromoendoscopy [FICE]) and confocal laser endomicroscopy (CLE) have been used for optical differentiation between neoplastic and non-neoplastic early colorectal lesions. All these techniques rely on single point in time optical diagnosis of the lesions and have provided variable levels of accuracy.

Therefore there remains a need for a less invasive and more accurate method for determining the benign or malignant state of a suspect bodily tissue such as a lesion in order to improve the outcome of treatment for patients.

Poellinger et al (Breast Cancer: Early- and Late- Fluorescence Near-Infrared Imaging with Indocyanine Green - A Preliminary Study; Radiology, Volume 258: Number 2, Feb 2011) describes a study investigating early- and late-fluorescence near-infrared imaging corresponding to the vascular and extravascular phases of indocyanine green enhancement for breast cancer detection and benign versus malignant breast lesion differentiation. The study indicates that early- and late-fluorescence ratio imaging after indocyanine green administration can be used to distinguish malignant from benign breast lesions.

Schmidt et al (Feasibility of real-time near-infrared indocyanine green fluorescence endoscopy for the evaluation of mucosal head and neck lesions; Head & Neck, February 2017, 234-240) describes a study exploring the feasibility of near-infrared endoscopy with indocyanine green to examine mucosal head and neck lesions. Such endoscopy was shown to be feasible and safe in the subjects and may be able to differentiate benign from malignant lesions.

Kumar et al (Fluorescence lifetime-based contrast enhancement of indocyanine green-labelled tumors; Journal of Biomedical Optics, 22(4), 2017) describes the use of indocyanine-green in conjunction with fluorescence lifetime detection to identify tumour-tissue in mouse models of subcutaneous human breast and brain tumours.

Onda et al (Preferential tumor cellular uptake and retention of indocyanine green for in vivo tumor imaging; Int. J. Cancer: 139, 673-682 (2016)) describes a study examining the mechanism of tumour imaging using intravenously administered indocyanine green. Indocyanine green fluorescence imaging was detected in xenograft tumours, based on their preferential cellular update and retention of the dye.

Nagahara et al (Fluorescence tumor imaging by i.v. administered indocyanine green in a mouse model of colitis-associated colon cancer; Cancer Science, 2018, 109, 1638-1647) investigates the tumour imaging capability and imaging mechanism of i.v. administered indocyanine green in a mouse model of colitis-associated colon cancer. The study indicates that fluorescence contrast-enhanced imaging following i.v. administer indocyanine green can be used to detect colon tumours in a mouse colitis-associated colon cancer model.

Jiang et al (Optimization of the enhanced permeability and retention effect for near-infrared imaging of solid tumors with indocyanine green; Am J Nucl Med Mol Imaging, 2015; 5(4); 390-400) describes a study investigating the optimal time and dose for imaging solid tumours using indocyanine green.

Onda et al (Fluorescence contrast-enhanced proliferative lesion imaging by enema administration of indocyanine green in a rat model of colon carcinogenesis; Oncotarget, 2017, Col. 8, No, 52, 90278-90290) describes a study which investigates indocyanine green availability in fluorescence imaging of the colon to identify proliferative lesions during colonoscopy. The results suggested that fluorescence contrast-enhanced imaging following the administration of an indocyanine green containing enema can enhance the detection of mucosal proliferative lesions of the colon during colonoscopy.

### Summary of the Invention

It is one aim of the present invention, amongst others, to provide a method of analysing a target bodily tissue of a subject and a device for use in such a method which address at least one disadvantage of the prior art, whether identified here or elsewhere, or to provide an alternative to existing methods and devices. For instance it may be an aim of the present invention to provide a method which can assist in the accurate determination of the malignant or benign state of a tissue, for example a rectal lesion.

According to aspects of the present invention, there is provided a method and device as set forth in the appended claims. Other features of the invention will be apparent from the dependent claims, and the description which follows.

According to a first aspect of the present invention, there is provided a method of analysing a target bodily tissue of a subject, the method comprising the steps of:
a) receiving image data, in a data analysis unit, of the target bodily tissue and a background bodily tissue adjacent to the target bodily tissue, the image data being obtained over a time period after dosing the subject with a contrast agent;
b) determining an intensity of light emitted from a first area of the target bodily tissue and an intensity of light emitted from a first area of the background bodily tissue at a plurality of time points during the time period, the intensity of light emitted from the target bodily tissue and the background bodily tissue at each of the plurality of time points being dependent upon the amount or brightness of contrast agent present in said tissues at each time point;
c) comparing the intensity of light emitted from the first area of the target bodily tissue and the intensity of light emitted from the first area of the background bodily tissue over the plurality of time points;
d) determining for a first part of the time period and a second part of the time period, the first part of the time period being earlier in the time period than the second part of the time period,

wherein the first time period is an initial uptake phase of the contrast agent and wherein the second part of the time period is a washout phase of the contrast agent, whether:
   1) the intensity of light emitted from the target bodily tissue is lower than the intensity of light emitted from the background bodily tissue during the first part of the time period; and whether
   2) the intensity of light emitted from the target bodily tissue is higher than the intensity of light emitted from the background bodily tissue during the second part of the time period;
wherein a negative determination in relation to 1) and/or 2) is indicative of the target bodily tissue being benign and a positive determination in relation to 1) and/or 2) is indicative of the target bodily tissue being malignant; and
wherein step d) 2) involves determining whether the intensity of light emitted from the target bodily tissue and the intensity of light emitted from the background bodily tissue have a convergent or divergent relationship over the second part of the time period, wherein a convergent relationship is indicative of the target bodily tissue being benign and a divergent relationship is indicative of the target bodily tissue being malignant.

The inventors have found that the intensity of light emitted from a target bodily tissue is lower than the intensity of light emitted from the background bodily tissue during an initial time period shortly after administration of a suitable contrast agent to a subject (i.e. an initial uptake phase), if the target bodily tissue is malignant. The inventors have also found that the intensity of light emitted from the target bodily tissue is higher than the intensity of light emitted from the background bodily tissue in a later time period after administration of the contrast agent to the subject (i.e. a washout phase), if the target bodily tissue is malignant. These differences in light emitted from the target bodily tissue and the background bodily tissue may be due to the different amounts of contrast agent present in the different tissues at different times after administration or may be due to a localised increase in brightness of the contrast agent in the target bodily tissue due to some other mechanism.

These effects are believed to be due to the differing pharmacokinetic properties of malignant tissue structures to similar benign tissue structures or adjacent healthy tissue. Therefore these tissue types have been found to absorb and excrete compounds such as contrast agents at different rates, which provide different intensities of light emission at different time points after dosing of the contrast agent.

These two features of the light emission profiles of target bodily tissues can be used, either alone or in combination, to determine the benign or malignant state of a tissue. Step d) involves determining both 1) and 2). A positive determination in relation to 1) and 2) is indicative of the target bodily tissue being malignant and a negative determination in relation to 1) and 2) is indicative of the target bodily tissue being benign.

In this way, the method of this first aspect may provide a more efficient and more accurate determination of the benign or malignant state of a tissue compared to known methods. This may be useful for a clinician to take into account when deciding on the most appropriate course of action for the subject and may therefore lead to improved clinical outcomes for the subject. This method may also allow the clinician to avoid taking a biopsy and/or resecting the target bodily tissue, and therefore avoid the complications which can occur with these procedures. The method may also reduce the number of surgical interventions performed on such patients that ultimately prove to have been unnecessary and therefore provide overall cost savings in the management of relevant health conditions.

Suitably the subject is a human or animal, suitably a human. The subject may be alternatively referred to as a patient. The subject may be suspected of having a cancerous growth on or in their body. For example, the subject may have presented with one or more symptoms of colorectal cancer. The subject may have a lesion in their gastrointestinal tract which may be benign or malignant and this state may not be determinable by simple observation of the lesion.

The target bodily tissue may be a polyp, growth, tumour or lesion, suitably a lesion. In some embodiments, the target bodily tissue may be located in the gastro-intestinal tract of the subject, suitably in the rectum, colon, duodenum or oesophagus of the subject.

In some embodiments the target bodily tissue is a rectal lesion.

The background bodily tissue adjacent to the target bodily tissue is suitably a healthy tissue in the same area of the subject's body, for example a healthy area of the rectum adjacent to a rectal lesion. Suitably the background bodily tissue is the same tissue type as the target bodily tissue. Suitably the background bodily tissue is close enough to the target bodily tissue for both the background bodily tissue and the target bodily tissue to appear within the same viewing field, for instance a single frame of an image data input taken with a suitable visualisation device, for example an endoscope.

The observation occurring in step a) on which the method of this first aspect is based is made over a time period after administration of a contrast agent to the subject. Suitably the time period over which the image data is obtained starts at substantially the same time or shortly after the administration of the contrast agent to the subject. The time period may begin shortly before the administration of the contrast agent to the subject in order to ensure that the target and background tissues are imaged when the contrast agent first reaches these tissues. Suitably the time period starts within 1 minute of dosing the subject with the contrast agent.

The image data may be obtained by and received from a suitable visualisation device or devices placed on or inside the subject's body. Suitably the target tissue is located inside the subject's body and therefore the image data is obtained by a suitable visualisation device placed inside the subject's body. For example, the image data may be (or have been) obtained by an endoscope or a laparoscope. Suitably the image data is digital image data. In some embodiments the image data is received in step a) of the method as it is being obtained by a suitable visualisation device, therefore in "real time". In some embodiments, the image data may have been recorded onto a suitable recording device and step a) involves receiving or accessing the image data recording, suitably from said recording device.

Suitably the image data comprises a field of view which is relatively static throughout the duration over which the image data is obtained, the field of view including the target bodily tissue and the background bodily tissue. This static field of view facilitates analysis of the same parts of the target bodily tissue and the background bodily tissue during the method.

In order to obtain the image data of the target bodily tissue and the background bodily tissue, the target bodily tissue and the background bodily tissue are suitably illuminated, suitably with visible light, during the time period. In order to make use of certain contrast agents dosed to the subject, the target bodily tissue and the background bodily tissue is suitably irradiated with another suitable form of electromagnetic radiation besides visible light.

In some embodiments, the image data is/was obtained whilst the target bodily tissue and the background bodily tissue are/were irradiated with near infrared light. Suitably the image data is/was obtained whilst the target bodily tissue and the background bodily tissue are/were illuminated with visible light and irradiated with near infrared light. Near infrared light (NIR) can penetrate bodily tissues and excite a fluorophore in a contrast agent present in the tissue, without causing autofluorescence of the tissue itself. Therefore near infrared light may be particularly useful wherein the contrast agent comprises a suitable fluorophore.

Suitably the contrast agent dosed to the subject during the video recording is indocyanine green. Indocyanine green (ICG) is a sterile, water-soluble but relatively hydrophobic tricarbocyanine molecule. Following intravenous injection, ICG is rapidly bound to plasma proteins with minimal leakage into the interstitium and is excreted by the liver in bile about 8 min after injection.

Once a tumour lesion exceeds a few millimetres in diameter, hypoxia and nutrient deprivation triggers an 'angiogenic switch' to allow the tumour to progress. Driven predominantly by vascular endothelial growth factor, the new vasculature is leaky due to uncoupling of endothelial cell-cell junctions causing the extravasation of the ICG out of the vessels. Once in the stroma, there is preferential tumour uptake of ICG by endocytosis (inversely proportional to cellular tight junctions). Intracellular ICG is entrapped in the membrane traffic system resulting in its slow turnover and prolonged retention by tumour cells.

Indocyanine green comprises a fluorophore which is excited by near infrared light to produce a detectable difference in the light emitted from bodily tissues which contain different amounts of ICG at particular times. Therefore in some embodiments the image data of step a) is or has been obtained over a time period after dosing the subject with indocyanine green whilst the target bodily tissue and the background bodily tissue were irradiated with near infrared light and suitably also illuminated with visible light, preferably with a combination of near infrared light and visible light.

The image data received in step a) suitably comprises red, green and blue channel data (RGB data). Suitably the image data comprises infrared channel data. Suitably the image data comprises both RGB data and infrared channel data.

Suitably the image data comprises a series of frames containing RGB data (and therefore a depiction of the target bodily tissue and the background bodily tissue) which are fixed in relation to the position of the target bodily tissue and the background bodily tissue.

Suitably the time period over which the image data is obtained is at least 2 minutes long, suitably at least 5 minutes, suitably at least 10 minutes, suitably at least 15 minutes long. Suitably the time period is up to 1 hour long, suitably up to 45 minutes, suitably up to 30 minutes long. Suitably the time period is from 5 minutes to 1 hour long, suitably from 10 minutes to 45 minutes long, suitably from 20 minutes to 30 minutes long or from 25 minutes to 30 minutes long.

Step b) of the method involves determining an intensity of light emitted from a first area of the target bodily tissue and an intensity of light emitted from a first area of the background bodily tissue at a plurality of time points during the time period. The first area of the target bodily tissue and the first area of the background bodily tissue are suitably fixed throughout the time period and throughout the analysis of step b). Fixing these areas during the time period and the analysis insofar as possible may improve the accuracy of the data obtained. These areas are suitably selected by the operator and comprise a sufficiently wide field of view to include normal (background) and suspect (target) tissue. The areas are determined to be of interest as potential sites of cancer as determined by the observer due to some identifiable characterisation or distinctive feature raising concern or potential due to biological considerations that the area may contain malignant tissue. Suitably the target areas are in the order of 1-10 cm in diameter although may be larger or smaller.

Step b) of the method may involve determining an intensity of light emitted from a second area of the target bodily tissue and/or an intensity of light emitted from a second area of the background bodily tissue at a plurality of time points during the time period, in addition to the first areas of the target and background tissues. Step b) may also involve determining an intensity of light emitted from further areas of the target and background tissues. Taking such multiple intensity data from different areas of the tissues may provide improved accuracy and consistency of the results produced. Such intensity data from second and further areas of the target and background tissues is suitably processed in the same way as the data from the first areas of the target and bodily tissues described below.

Suitably the image data is received in RGB format and step b) involves combining the red, green and blue channel data for each pixel in the image data in the first area of the target bodily tissue and in the first area of the background bodily tissue to provide a grayscale format comprising a single data point for each pixel of each of the first areas of target and background tissues. Suitably this operation of combining the red, green and blue channel data is performed on a series of frames extracted from the image data at the plurality of time intervals during the time period. Suitably one frame of the image data is extracted per time interval and then analysed as described above. Alternatively, the image data may be received in any suitable digital format which allows the data processing of steps b) to d) to be carried out.

Suitably the plurality of time points are separated by intervals of 0.5 to 30 seconds, suitably by intervals of 0.5 to 5 seconds, suitably by intervals of 0.5 to 2 seconds. Suitably the plurality of time points are separated by approximately 1 second or by 1 second. Suitably the plurality of time points are separated by intervals of up to 120 seconds.

Step b) suitably provides an intensity of light emitted by the first area of the target bodily tissue and the first area of the background bodily tissue at each of the plurality of time intervals which can then be compared in step c) to determine the features of the intensities over the time period (as a surrogate indicator of the concentration of the contrast agent present in the tissues) and therefore to determine the malignant or benign state of the tissues.

Step d) involves determining whether relationships 1) and 2) are present for a first part of the time period and a second part of the time period, the first part of the time period being earlier in the time period than the second part of the time period. The first part is a period of initial uptake of the contrast agent in the tissues wherein the intensity of light emitted starts at a baseline level and rises as the contrast agent is taken up by the tissues.

The first part of the time period may start up to 1 minute into the time period, suitably up to 30 seconds into the time period, suitably at the start of the time period. Suitably the first part of the time period ends up to 5 minutes into the time period, suitably up to 3 minutes into the time period, suitably up to 2 minutes into the time period. Suitably the first part of the time period comprises the part of the time period from 0 minutes to 5 minutes into the time period, suitably from 0 minutes to 2 minutes into the time period. Suitably the first part of the time period starts at 0 minutes into the time period and ends at up to 3 minutes into the time period.

Suitably the times of the first part of the time period discussed above in relation to the time period also have the same or approximately the same relationship to the time at which the subject was dosed with the contrast agent.

The second part of the time period is a washout period wherein the concentration of the contrast agent declines from a high point as the contrast agent is removed or excreted from the tissues. Suitably the second part of the time period starts at least 5 minutes into the time period and/or suitably up to 10 minutes into the time period. Suitably the second part of the time period ends at least 15 minutes into the time period and/or suitably up to 40 minutes into the time period, suitably up to 35 minutes, suitably up to 30 minutes. Suitably the second part of the time period ends at the end of the time period. Suitably the second part of the time period starts at up to 25 minutes into the time period and ends at least 30 minutes into the time period. Suitably the second part of the time period starts at approximately 20 minutes into the time period and ends approximately 30 minutes into the time period.

The times of the second part of the time period discussed above in relation to the time period suitably also have the same or approximately the same relationship to the time at which the subject was dosed with the contrast agent.

Step d) may involve plotting a graphical representation of the intensity data for the target bodily tissue and the background bodily tissue in order to determine the relationships 1) and 2) of step d). Step d) may also involve applying a logarithmic operation to the intensity data on the graphical representation in order to account for outliers in the data and to more clearly show whether the relationships 1) and 2) are present. These operations may further assist a clinician in deciding on a determination of the target bodily tissue. While plots are discernible as distinctive by apparent trend differentials, additional features of relevance are likely to be made evident by more sophisticated analysis.

Step d) 2) involves determining whether the intensity of light emitted from the target bodily tissue is higher than the intensity of light emitted from the background bodily tissue during the second part of the time period. In a graphical representation of the intensity data for the target bodily tissue and the background bodily tissue discussed above, this relationship may manifest in a plot of the intensities of the target and background tissue either diverging or converging during the time period, particularly in the second part of the time period. The inventors have found that a diverging relationship is indicative of a malignant state of the target bodily tissue and a convergent relationship is indicative of a benign state of the target bodily tissue. A diverging relationship between the intensities of the target and background tissue may be indicated by the plot of the relative intensities being separated by a significant value at the end of the time period. Suitably such differences in intensity are larger than the expected usual variance in intensities of each tissue. A converging relationship between the intensities of the target and background tissue may be indicated by the plot of the relative intensities being separated by an insignificant value and/or being approximately the same at the end of the time period.

Therefore step d) 2) involves determining whether the intensity of light emitted from the target bodily tissue and the intensity of light emitted from the background bodily tissue have a convergent or divergent relationship over the second part of the time period, wherein a convergent relationship is indicative of the target bodily tissue being benign and a divergent relationship is indicative of the target bodily tissue being malignant.

Suitably, step d) is completed within an appropriate time frame that allows direction of an interventional procedure or which allows direction of subsequent treatments and other follow-up strategies.

In some embodiments, the method of this first aspect may involve, in step a), receiving the image data obtained over a time period after dosing the subject with more than one contrast agent, suitably wherein the different contrast agents emit distinctly different colours (or wavelengths) of light.

In some embodiments, the method of this first aspect may involve repeating steps a) to d) using a second contrast agent, suitably wherein the second contrast agent emits distinctly different colours (or wavelengths) of light compared to the contrast agent dosed to the subject in order to carry out the first sequence of steps a) to d).

In some embodiments, the method of this first aspect may involve repeating steps a) to d) using image data obtained from a viewing angle with respect to the target and background tissue different to the viewing angle of the image data obtained in the first sequence of steps a) to d).

In some embodiments, the method of this first aspect may involve carrying out steps a) to d) simultaneously using image data obtained from at least two different viewing angles with respect to the target and background tissue, for example using at least two different visualisation devices.

Also described is a method of analysing a target bodily tissue of a subject, the method comprising the steps of:
a) receiving an image data recording of the target bodily tissue and a background bodily tissue adjacent to the target bodily tissue, the image data recording having been obtained over a time period after dosing the subject with a contrast agent;
b) determining an intensity of light emitted from a first area of the target bodily tissue and an intensity of light emitted from a first area of the background bodily tissue at a plurality of time points during the time period, the intensity of light emitted from the target bodily tissue and the background bodily tissue at each of the plurality of time points being dependent upon the amount or brightness of contrast agent present in said tissues at each time point;
c) comparing the intensity of light emitted from the first area of the target bodily tissue and the intensity of light emitted from the first area of the background bodily tissue over the plurality of time points;
d) determining for a first part of the time period and a second part of the time period, the first part of the time period being earlier in the time period than the second part of the time period, wherein the first time period is an initial uptake phase of the contrast agent and wherein the second part of the time period is a washout phase of the contrast agent, whether:
   1) the intensity of light emitted from the target bodily tissue is lower than the intensity of light emitted from the background bodily tissue during the first part of the time period; and
   2) the intensity of light emitted from the target bodily tissue is higher than the intensity of light emitted from the background bodily tissue during the second part of the time period;

wherein a negative determination in relation to 1) and/or 2) is indicative of the target bodily tissue being benign and a positive determination in relation to 1) and/or 2) is indicative of the target bodily tissue being malignant; and
wherein step d) 2) involves determining whether the intensity of light emitted from the target bodily tissue and the intensity of light emitted from the background bodily tissue have a convergent or divergent relationship over the second part of the time period, wherein a convergent relationship is indicative of the target bodily tissue being benign and a divergent relationship is indicative of the target bodily tissue being malignant.

The method of this further aspect may have any of the suitable features and advantages of the method of the first aspect.

According to a second aspect of the present invention, there is provided a device for analysing a target bodily tissue of a subject, the device comprising a visual inspection unit and a data analysis unit;
wherein the visual inspection unit comprises a visible light source and an image data capture unit; and
wherein the data analysis unit is adapted to:
   a) receive, from the image data capture unit, image data of said target bodily tissue and said background bodily tissue adjacent to said target bodily tissue, said image data taken over a time period;
   b) determine an intensity of light emitted from a first area of said target bodily tissue and an intensity of light emitted from a first area of said background bodily tissue at a plurality of time points of said time period; and
   c) compare said intensity of light emitted from said first area of said target bodily tissue and said intensity of light emitted from said first area of said background bodily tissue over said plurality of time points; and
   d) determine whether:
      1) said intensity of light emitted from said target bodily tissue is lower than said intensity of light emitted from said background bodily tissue during a first part of said time period; and
      2) said intensity of light emitted from said target bodily tissue is higher than said intensity of light emitted from said background bodily tissue during a second part of said time period;
wherein d) 2) involves determining whether the intensity of light emitted from the target bodily tissue and the intensity of light emitted from the background bodily tissue have a convergent or divergent relationship over the second part of the time period.

The device of this second aspect may have or embody any of the suitable features and advantages of the method of the first aspect.

Suitably the data analysis unit is adapted to carry out a method of the first aspect.

The data analysis unit is suitably adapted to carry out the stated operations by the provision of suitable software, examples of which are provided in the detailed description below.

The visual inspection unit of the device of this second aspect may be an endoscope or a laparoscope. Suitably the visual inspection unit is an endoscope.

Suitably the visual inspection unit comprises a near infra-red light source.

According to a third aspect of the present invention, there is provided a computer readable medium comprising code configured to implement the method of the first aspect of the invention and/or to implement the operations of the data analysis of the device of the second aspect. The device of the second aspect, the data analysis unit in particular, may comprise or use the computer readable medium. The computer readable medium may be a non transitory computer readable medium.

There is also provided a computer program configured (e.g. coded) to implement the method of the first aspect of the invention and/or to implement the operations of the data analysis unit of the device of the second aspect. The device of the second aspect may comprise or use the computer program.

### Brief Description Of The Drawings

For a better understanding of the invention, and to show how example embodiments may be carried into effect, reference will now be made to the accompanying drawings in which:
Figure 1 shows an example of the view of the image data received in step a) of the method of the first aspect.
Figures 2 and 3 show graphs comparing the surface to background ratios of light intensity from malignant and benign tumours.
Figure 4 shows a graph of light intensity from a rectal polyp and background healthy tissue during an initial phase of the time period.
Figure 5 shows a graph of light intensity from a rectal cancer and background healthy tissue during an initial phase of the time period.
Figure 6 shows a graph of light intensity from a benign rectal polyp and background healthy tissue, over 25 min.
Figure 7 shows a graph of light intensity from a rectal cancer and background healthy tissue.

### Detailed Description Of The Example Embodiments

### Method

16 adult male and female patients with suspected rectal tumours were recruited onto a study involving the method and device of the present invention. The suspected rectal tumours had previously been diagnosed after endoscopy with or without biopsies. Patients with any history of allergic reaction to iodine were excluded.

The rectal lesions present in these patients were visualized transanally by using the pinpoint Novadaq laparoscope under NIR light. The method of visualisation varied depending on the location of the suspected tumour. For low rectal lesions, a lone star, a proctoscope or an Eisenhammer speculum was used for optimal visualisation. For high rectal lesions a rigid sigmoidoscope or TAMIS port was used.

Once the lesion was visualised and the setup was complete, 0.25 mg per kg body weight of patient of ICG contrast agent was injected intravenously and video (image data) recorded in the NIR mode. The video recording taken consisted of real colour, infrared and composite images. During the recording, the lesion (suspected tumour) target tissue and the background tissue adjacent to the lesion were both visible in the same frame for the purpose of analysis. The setup for the video recording was very important. It was necessary to ensure that stable clear views of both the lesion and the healthy background tissue were maintained throughout. Any fluid like mucus or blood gave a spurious reading so every attempt was made to keep the field of view dry. This was made possible with an ENT suction tube with a narrow diameter allowing insertion along the 10 mm Novadaq laparoscope through the rigid sigmoidoscope so as to get uninterrupted video data. Video was recorded for 25 to 30 minutes from the time of injection of ICG.

At the time of taking the video recording, note was made of the initial uptake of ICG by the lesion versus the background tissue and the washout of the florescence (and therefore the ICG) at 25 to 30 minutes to reach a provisional determination of the malignant or benign state of the target tissue. However, in around 50 % of the cases these differences were very subtle so could not be determined by eye. Therefore the intensity of the light emitted by the tissue, and therefore recorded in the video recording, was measured using Imaged and Mat Lab software. The data analysis comprised the following steps:

### 1. Data extraction

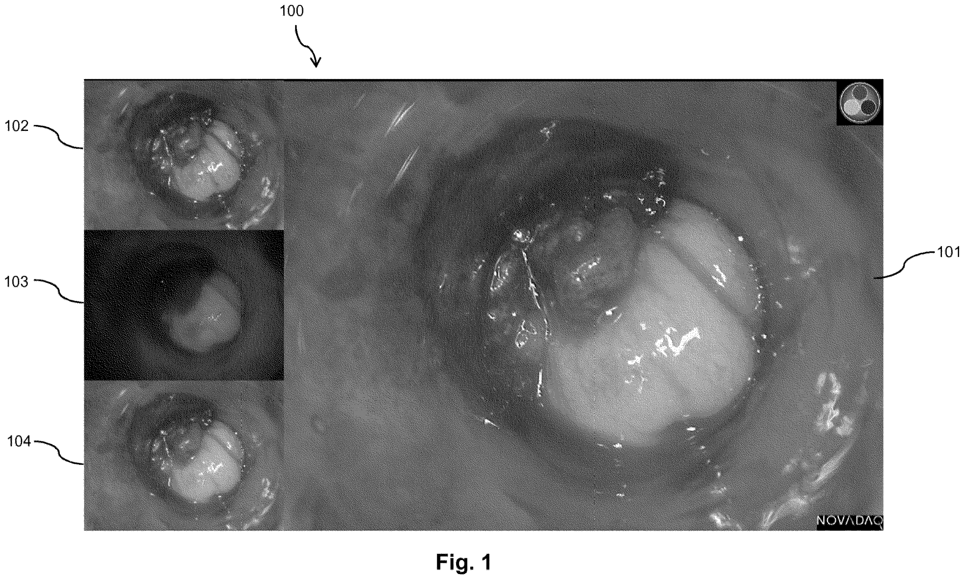

The video recording data (100, Figure 1) consisted of a visible light image thumbnail (102), an infrared image thumbnail (103) and a false colour composite image thumbnail (104), with each image available to be shown in real time in the larger main display image (101). This data is captured during the procedure and the type of main display image can be selected based on the needs of the surgeon during the investigation, so the analysis was performed on the thumbnail images which remain available throughout the video dataset, irrespective of the type of main display image.

The captured video data was saved in MP4 (.mp4) format or as a number of datasets stitched together in QuickTime format (.mov). The framerate of the video recording was typically 29 frames/second. These images were analysed using the FIJI image processing software alongside MATLAB software for some pre-processing tasks.

In order to reduce the number of frames and expedite analysis in the FIJI software, MATLAB was used to extract one frame from each second of the video recording. This was done using the Video Reader and Video Writer functions of MATLAB. This resulted in 1500-2000 frames per analysis, which corresponds to about 25-30 minutes of video recording. The resultant selected video recording data was saved in an uncompressed AVI (.avi) format to allow for easy import into the FIJI software.

The original video recording data was displayed in an RGB format, which has three different colour channels corresponding to red (R), green (G) and blue (B). Each of these colour channels was segmented into 256 discrete levels (0-255) corresponding to shades of the channel colour. These shades are the various amounts of light which have fallen on the RGB pixels of the video recording equipment. The video recording data was then copied and converted to grayscale data to allow for intensity analysis of the infrared thumbnail image. For further ease of analysis the image was also cropped to the size of the thumbnail at this point.

In converting the video recording data to a grayscale format, the 256 discrete levels were preserved but converted to a weighted sum of the three channels (RGB) into another channel. This summed channel gives the total amount or intensity of light which has fallen on each pixel (and therefore corresponds to the light emitted from each specific area of the target and background tissues). The light levels, and hence the concentration of ICG, can then be represented by a single value of grayscale rather than by three values of the RGB colour.

FIJI software was used to open the original video recording data and the cropped grayscale data and synchronise the actions between these windows. A point measurement tool was then placed over a region of target tissue and a region of healthy background tissue in each frame, and the measurements of these areas recorded.

As the intensity image (the grayscale image) and the original image have been synchronised, this allowed the target tissue to be identified in the original image and the measurements of the intensity image to be taken from the same position as identified in the original image. The placements of all point measurements were made with the consensus of a medical physicist and a surgical doctor experienced in this field for the whole duration of the video.

The relative measurements obtained in this manner allowed the use of these measurements without the need for a calibration for contrast agent concentration, as it gave only the difference between the contrast agent concentration in the tumour and the healthy background, not the absolute measure. The use of a point measurement also allowed the analysis to be independent of variations in size of the target tissue.

### 2. Data Analytics

The relative intensity data was exported from FIJI as a Comma Space Variable (.csv) file. This file can be imported into Microsoft Excel or equivalent. The data was plotted as a scatter chart with the image frame number on the x-axis (this corresponds to the time point the measurement was taken) and the pixel value on the y-axis (this corresponds to the intensity of the ICG, and therefore corresponds to its concentration). The pixel or intensity values can only be compared if they have been measured in the same frame.

The data was fitted with a logarithmic curve; this can be done using the trend fitting function which is available in Microsoft Excel. Logarithmic curves are used to smoothen variations or outliers in the data and to provide a pattern of intensity trace. Ratio data was obtained by comparing the intensity values from the same frame for both the target and the healthy tissue. Mean intensities and target tissue to background ratios were also compared both before resection and in ex vivo samples post resection.

A provisional determination of the malignant or benign state of the suspected rectal tumour tissue for all patients was made based on the video recording data analysis and compared with a histological analysis which was performed post resection.

The intensity data was then analysed to reach a provisional diagnosis which is compared to the histological diagnosis post resection.

### Results

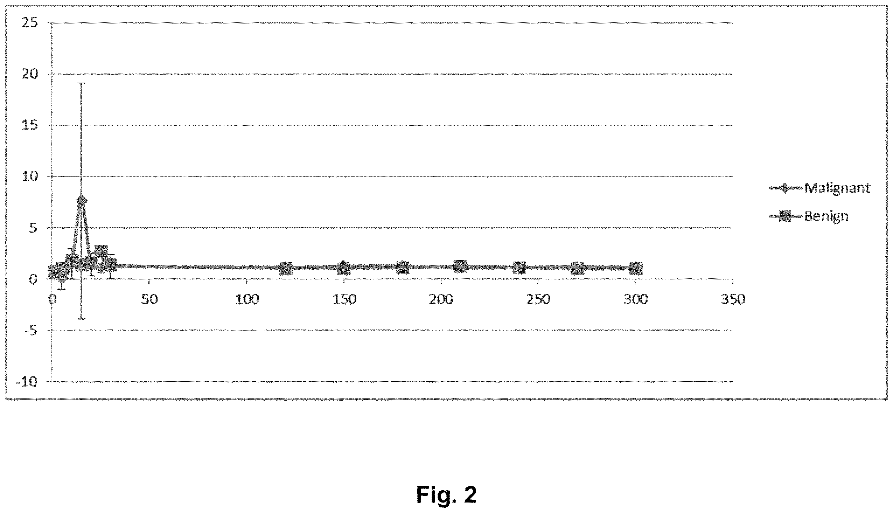

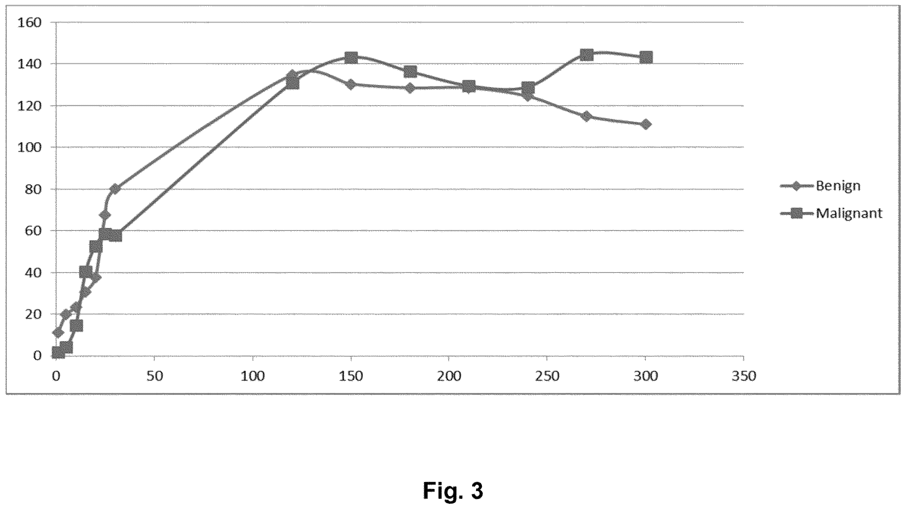

Single point in time variables like absolute or mean intensities and surface to background ratios (SBR) were explored for their discriminatory information. However, these single time values could not differentiate the benign lesions from cancers (see Figures 2 and 3).

The method described above, which may be described as a dynamic visual analysis (DVA) which involved determining the relationship between the dynamic intensity traces of lesion and healthy background tissue, showed consistency in differentiating the cancers from the benign lesions.

The following dynamic variables were used in the differentiation.

### A. Initial uptake

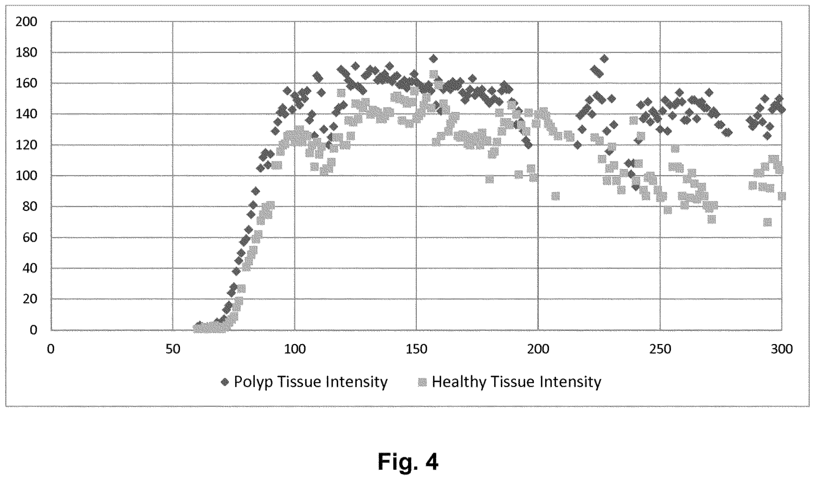

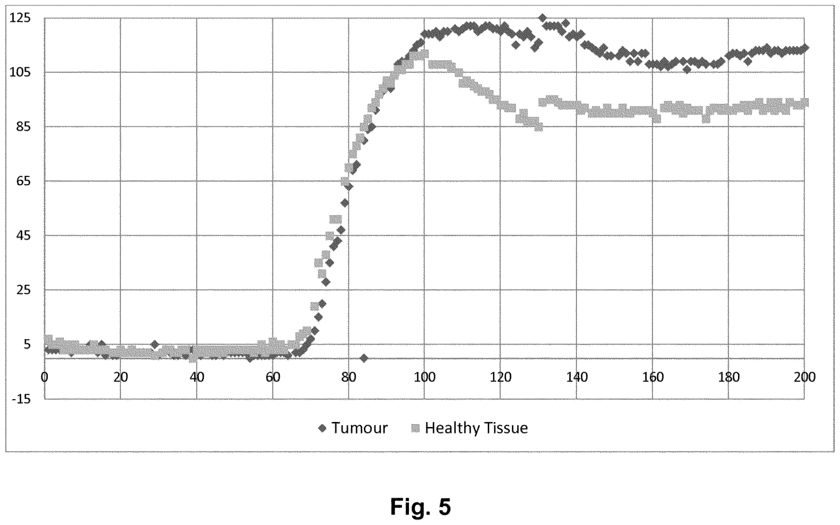

We observed that the initial uptake of the ICG contrast agent in benign lesions was either synchronous with the background tissue or was ahead of the background tissue. These observations were confirmed graphically by using the MATLAB and imaged (Figure 4). Figure 4 shows the intensity trace of a rectal polyp for 300 seconds (i.e. an initial phase or "first part" of the time period over which the video recording has been made). The time in seconds is plotted on the x-axis and the florescence (light) intensity on the y-axis. The polyp is actually more fluorescent than the normal background tissue from the beginning and there is no lag in the uptake of the ICG. In cancerous tissue, there was a definite lag in the initial uptake of the ICG. This observation was often difficult to appreciate by the human eye but was much clearer once graphically plotted (Figure 5). Figure 5 shows the intensity data of a rectal cancer over 200 seconds as compared to normal background tissue. The y-axis gives the light intensity while the x-axis is the time in seconds. The cancer lags in the uptake of ICG initially as evident by the intensity trace.

### B. Washout of contrast agent

At 25 to 30 minutes most of the ICG tends to washout of benign lesions while cancerous tissue retains it for longer.

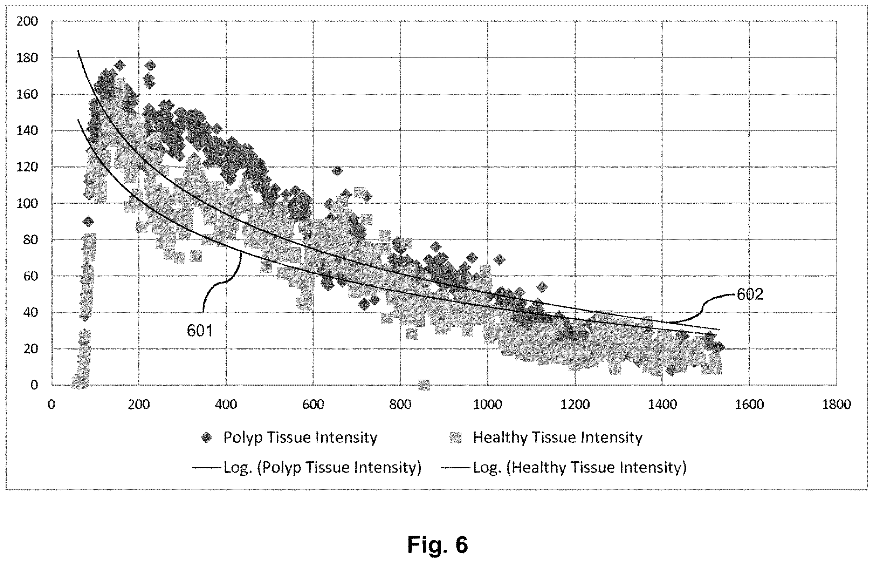

We noted some disparity between observers while commenting on the washout phase (i.e. a "second part" of the time period of the video recording) at 25-30 minutes whether the lesions were still florescent or not. Therefore the relative intensities had to be quantified. The whole intensity trace of the lesion and the background tissue were plotted together. After inserting the logarithmic trend lines to exclude the outliers, further discriminatory information was extracted. In benign lesions the trend lines were convergent over time, particularly during the "washout" period, and even crossed in some patients. Figure 6 shows the intensity trace of a benign rectal polyp over 25 min. There is hardly any difference in the intensity between the polyp (line 602) and healthy tissue (line 601) at 25 minutes and the trend lines are convergent over time.

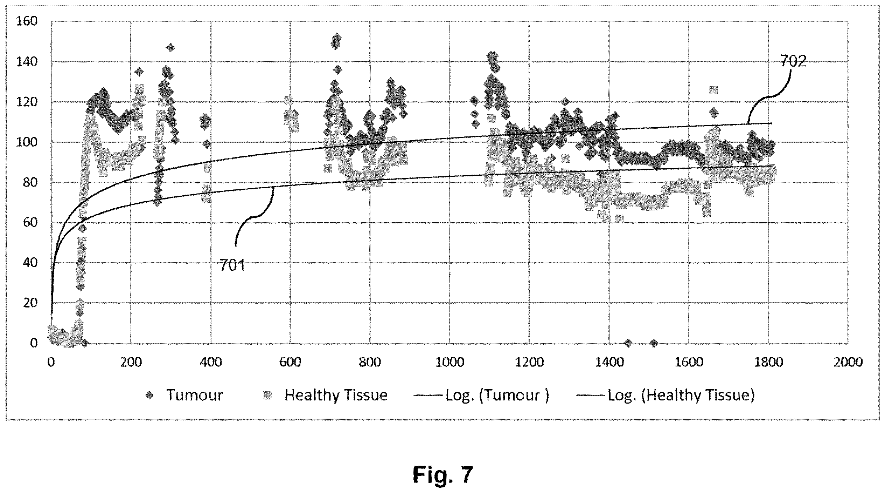

In cancerous tissue, the trend lines were actually divergent (see Figure 7). Figure 7 shows the complete intensity trace of a rectal cancer in comparison with the background healthy tissue showing the differential retention of the ICG at 25-30 minutes. The trend lines are divergent over time.

The provisional diagnosis after the DVA was based on at least 1 variable being confidently reported. One case was excluded as the video recording lacked the early uptake part and there were no data points recorded in the latter part of the video recording to confidently analyse and make a determination. In 2 cases the initial few seconds of the uptake was not recorded but the complete 25-30 minutes video was available for analysis and the diagnosis was based on the trend lines. Also in 2 patients only the initial uptake was confidently recorded so the diagnosis was based on whether there was a difference in early uptake.

### Histological comparison

Based on the dynamic video analysis discussed above, of the 15 patients, 8 lesions were determined as benign and then confirmed as benign by histology post resection. The other 7 lesions were determined as being cancerous, which was again confirmed by histology post resection.

In 3 of the patients, the dynamic video analysis was performed pre and post chemo radiotherapy (CRT). In one of the post CRT patients, only the initial uptake was confidently recorded so the diagnosis was based on this uptake data only. In all 3 of these patients, the post CRT analysis confirmed the target tissue was cancerous meaning these patients were not suitable for a "watch and wait" option and were sent for radical resection of the target tissue. Histology confirmed residual cancer in all 3 cases.

These results show that the real time optical determination of the malignant or benign state of a suspected target tissue is possible by the method of the present invention. Implementing this method into wider clinical practice may therefore save considerable cost, time and patient morbidity due to the ease, speed and accuracy of the method compared to current methods and by allowing a clinician to only treat suspected cancerous tissue when necessary.

In summary, the present invention provides a method of analysing a target bodily tissue of a subject. The method involves manipulating image data from the target bodily tissue and a background bodily tissue after dosing the subject with a contrast agent. The relative intensities of the light emitted from selected areas of the target bodily tissue and background bodily tissue at different time points after dosing are compared to determine whether 1) the intensity of light emitted from the target bodily tissue is lower than the intensity of light emitted from the background bodily tissue during the first part of the time period; and/or 2) the intensity of light emitted from the target bodily tissue is higher than the intensity of light emitted from the background bodily tissue during the second part of the time period; to assist in the determination of whether the target bodily tissue is benign or malignant.

Throughout this specification, the term "comprising" or "comprises" means including the component(s) specified but not to the exclusion of the presence of other components. The term "consisting essentially of' or "consists essentially of' means including the components specified but excluding other components except for materials present as impurities, unavoidable materials present as a result of processes used to provide the components, and components added for a purpose other than achieving the technical effect of the invention.

The term "consisting of' or "consists of" means including the components specified but excluding addition of other components.

Whenever appropriate, depending upon the context, the use of the term "comprises" or "comprising" may also be taken to encompass or include the meaning "consists essentially of" or "consisting essentially of', and may also be taken to include the meaning "consists of" or "consisting of'.

## Claims

1. A method of analysing a target bodily tissue of a subject, the method comprising the steps of:
a) receiving, in a data analysis unit, image data of the target bodily tissue and a background bodily tissue adjacent to the target bodily tissue, the image data being obtained over a time period after dosing the subject with a contrast agent;
b) determining an intensity of light emitted from a first area of the target bodily tissue and an intensity of light emitted from a first area of the background bodily tissue at a plurality of time points during the time period, the intensity of light emitted from the target bodily tissue and the background bodily tissue at each of the plurality of time points being dependent upon the amount or brightness of contrast agent present in said tissues at each time point;
c) comparing the intensity of light emitted from the first area of the target bodily tissue and the intensity of light emitted from the first area of the background bodily tissue over the plurality of time points;
d) determining for a first part of the time period and a second part of the time period, the first part of the time period being earlier in the time period than the second part of the time period, wherein the first time period is an initial uptake phase of the contrast agent and wherein the second part of the time period is a washout phase of the contrast agent, whether:
1) the intensity of light emitted from the target bodily tissue is lower than the intensity of light emitted from the background bodily tissue during the first part of the time period; and
2) the intensity of light emitted from the target bodily tissue is higher than the intensity of light emitted from the background bodily tissue during the second part of the time period;
wherein a negative determination in relation to 1) and/or 2) is indicative of the target bodily tissue being benign and a positive determination in relation to 1) and/or 2) is indicative of the target bodily tissue being malignant; and
wherein step d) 2) involves determining whether the intensity of light emitted from the target bodily tissue and the intensity of light emitted from the background bodily tissue have a convergent or divergent relationship over the second part of the time period, wherein a convergent relationship is indicative of the target bodily tissue being benign and a divergent relationship is indicative of the target bodily tissue being malignant.

2. The method according to claim 1, wherein the target bodily tissue is a rectal lesion.

3. The method according to claim 1 or claim 2, wherein the image data was obtained whilst the target bodily tissue and the background bodily tissue were irradiated with near infrared light.

4. The method according to any one of the preceding claims, wherein the time period is at least 5 minutes long.

5. The method according to any one of the preceding claims, wherein the contrast agent is indocyanine green.

6. The method according to any one of the preceding claims, wherein the image data is received in RGB format and step b) involves combining red, green and blue channel data for each pixel in the video recording in the first area of the target bodily tissue and in the first area of the background bodily tissue to provide a grayscale format comprising a single data point for each pixel.

7. The method according to any one of the preceding claims, wherein the plurality of time points are separated by intervals of up to 120 seconds.

8. The method according to any one of the preceding claims, wherein the first part of the time period starts at 0 minutes into the time period and ends at up to 2 minutes into the time period.

9. The method according to any one of the preceding claims, wherein the second part of the time period starts at up to 25 minutes into the time period and ends at least 30 minutes into the time period.

10. A device for analysing a target bodily tissue of a subject, the device comprising a visual inspection unit and a data analysis unit;
wherein the visual inspection unit comprises a visible light source and an image data capture unit; and
wherein the data analysis unit is adapted to:
a) receive, from the image data capture unit, image data of said target bodily tissue and said background bodily tissue adjacent to said target bodily tissue, said image data taken over a time period;
b) determine an intensity of light emitted from a first area of said target bodily tissue and an intensity of light emitted from a first area of said background bodily tissue at a plurality of time points of said time period;
c) compare said intensity of light emitted from said first area of said target bodily tissue and said intensity of light emitted from said first area of said background bodily tissue over said plurality of time points; and
d) determine whether:
1) said intensity of light emitted from said target bodily tissue is lower than said intensity of light emitted from said background bodily tissue during a first part of said time period; and
2) said intensity of light emitted from said target bodily tissue is higher than said intensity of light emitted from said background bodily tissue during said second part of said time period;
wherein d) 2) involves determining whether the intensity of light emitted from the target bodily tissue and the intensity of light emitted from the background bodily tissue have a convergent or divergent relationship over the second part of the time period.

11. The device according to claim 10, wherein the visual inspection unit is an endoscope.

12. The device according to claim 10 or claim 11, wherein the visual inspection unit comprises a near infra-red light source.

13. A computer readable medium comprising code configured to implement the method according to any one of claims 1 to 9 and/or to implement the operations of the data analysis of the device according to any one of claims 9 to 12.

14. A computer program which is configured, when executed in a computer, to implement the method according to any one of claims 1 to 9 and/or to implement the operations of the data analysis unit of the device according to any one of claims 9 to 12.

## Patentansprüche

1. Verfahren zur Analyse eines Ziel-Körpergewebes einer Testperson, wobei das Verfahren die folgenden Schritte umfasst:
a) Empfangen, in einer Datenanalyseeinheit, von Bilddaten des Ziel-Körpergewebes und eines Hintergrund-Körpergewebes, das an das Ziel-Körpergewebe angrenzt, wobei die Bilddaten über einen Zeitraum nach Verabreichung eines Kontrastmittels an die Testperson empfangen werden;
b) Bestimmen einer Intensität von Licht, das von einem ersten Bereich des Ziel-Körpergewebes emittiert wird, und einer Intensität von Licht, das von einem ersten Bereich des Hintergrund-Körpergewebes zu einer Vielzahl von Zeitpunkten während des Zeitraums emittiert wird, wobei die Intensität von Licht, das von dem Ziel-Körpergewebe und dem Hintergrund-Körpergewebe zu jedem der Vielzahl von Zeitpunkten emittiert wird, von der Menge oder Helligkeit des Kontrastmittels abhängt, das in den Geweben zu jedem Zeitpunkt vorhanden ist;
c) Vergleichen der Intensität des von dem ersten Bereich des Ziel-Körpergewebes emittierten Lichts und der Intensität des von dem ersten Bereich des Hintergrund-Körpergewebes emittierten Lichts über die Vielzahl der Zeitpunkte;
d) Bestimmen für einen ersten Teil des Zeitraums und einen zweiten Teil des Zeitraums, wobei der erste Teil des Zeitraums früher im Zeitraum liegt als der zweite Teil des Zeitraums, wobei der erste Zeitraum eine anfängliche Aufnahmephase des Kontrastmittels ist und wobei der zweite Teil des Zeitraums eine Auswaschphase des Kontrastmittels ist, ob:
1) die Intensität des von dem Zielkörpergewebe emittierten Lichts geringer ist als die Intensität des von dem Hintergrundkörpergewebe emittierten Lichts während des ersten Teils des Zeitraums; und
2) die Intensität des vom Ziel-Körpergewebe emittierten Lichts höher ist als die Intensität des vom Hintergrund-Körpergewebe emittierten Lichts während des zweiten Teils des Zeitraums;
wobei eine negative Bestimmung in Bezug auf 1) und/oder 2) angibt, dass das Ziel-Körpergewebe gutartig ist, und eine positive Bestimmung in Bezug auf 1) und/oder 2) angibt, dass das Ziel-Körpergewebe bösartig ist; und wobei Schritt d) 2) das Bestimmen beinhaltet, ob die Intensität des von dem Ziel-Körpergewebe emittierten Lichts und die Intensität des von dem Hintergrund-Körpergewebe emittierten Lichts über den zweiten Teil des Zeitraums ein konvergentes oder divergentes Verhältnis aufweisen, wobei ein konvergentes Verhältnis anzeigt, dass das Ziel-Körpergewebe gutartig ist, und ein divergentes Verhältnis anzeigt, dass das Ziel-Körpergewebe bösartig ist.

2. Verfahren nach Anspruch 1, wobei das Ziel-Körpergewebe eine rektale Läsion ist.

3. Verfahren nach Anspruch 1 oder Anspruch 2, wobei die Bilddaten erhalten wurden, während das Ziel-Körpergewebe und das Hintergrund-Körpergewebe mit Nahinfrarotlicht bestrahlt wurden.

4. Verfahren nach einem der vorhergehenden Ansprüche, wobei der Zeitraum mindestens 5 Minuten lang ist.

5. Verfahren nach einem der vorhergehenden Ansprüche, wobei das Kontrastmittel Indocyaningrün ist.

6. Verfahren nach einem der vorhergehenden Ansprüche, wobei die Bilddaten im RGB-Format empfangen werden und Schritt b) das Kombinieren von Rot-, Grün- und Blaukanaldaten für jedes Pixel in der Videoaufzeichnung im ersten Bereich des Ziel-Körpergewebes und im ersten Bereich des Hintergrund-Körpergewebes beinhaltet, um ein Graustufenformat bereitzustellen, das einen einzelnen Datenpunkt für jedes Pixel umfasst.

7. Verfahren nach einem der vorhergehenden Ansprüche, wobei die Vielzahl der Zeitpunkte durch Intervalle von bis zu 120 Sekunden getrennt sind.

8. Verfahren nach einem der vorhergehenden Ansprüche, wobei der erste Teil des Zeitraums bei 0 Minuten innerhalb des Zeitraums beginnt und bei bis zu 2 Minuten innerhalb des Zeitraums endet.

9. Verfahren nach einem der vorhergehenden Ansprüche, wobei der zweite Teil des Zeitraums nach bis zu 25 Minuten des Zeitraums beginnt und nach mindestens 30 Minuten des Zeitraums endet.

10. Vorrichtung zum Analysieren eines Ziel-Körpergewebes einer Testperson, wobei die Vorrichtung eine Sichtprüfungseinheit und eine Datenanalyseeinheit umfasst;
wobei die Sichtprüfungseinheit eine sichtbare Lichtquelle und eine Bilddatenerfassungseinheit umfasst; und
wobei die Datenanalyseeinheit für Folgendes ausgelegt ist:
a) Empfangen, von der Bilddatenerfassungseinheit, von Bilddaten des Ziel-Körpergewebes und des an das Ziel-Körpergewebe angrenzenden Hintergrund-Körpergewebes, wobei die Bilddaten über einen Zeitraum aufgenommen werden;
b) Bestimmen einer Intensität des von einem ersten Bereich des Ziel-Körpergewebes emittierten Lichts und einer Intensität des von einem ersten Bereich des Hintergrund-Körpergewebes emittierten Lichts zu einer Vielzahl von Zeitpunkten des genannten Zeitraums;
c) Vergleichen der Intensität des von dem ersten Bereich des Ziel-Körpergewebes emittierten Lichts und der Intensität des von dem ersten Bereich des Hintergrund-Körpergewebes emittierten Lichts über die Vielzahl von Zeitpunkten; und
d) Bestimmen, ob:
1) die Intensität des vom Ziel-Körpergewebe emittierten Lichts geringer ist als die Intensität des vom Hintergrund-Körpergewebe emittierten Lichts während eines ersten Teils des Zeitraums; und
2) die Intensität des vom Ziel-Körpergewebe emittierten Lichts höher ist als die Intensität des vom Hintergrund-Körpergewebe emittierten Lichts während des zweiten Teils des Zeitraums;
wobei d) 2) das Bestimmen beinhaltet, ob die Intensität des vom Ziel-Körpergewebe emittierten Lichts und die Intensität des vom Hintergrund-Körpergewebe emittierten Lichts über den zweiten Teil des Zeitraums eine konvergente oder divergente Beziehung aufweisen.

11. Vorrichtung nach Anspruch 10, wobei die Sichtprüfungseinheit ein Endoskop ist.

12. Vorrichtung nach Anspruch 10 oder Anspruch 11, wobei die Sichtprüfungseinheit eine Nahinfrarotlichtquelle umfasst.

13. Computerlesbares Medium, das Code umfasst, der ausgebildet ist, um das Verfahren nach einem der Ansprüche 1 bis 9 zu berechnen und/oder die Operationen der Datenanalyse der Vorrichtung nach einem der Ansprüche 9 bis 12 zu implementieren.

14. Computerprogramm, das, wenn es in einem Computer ausgeführt wird, ausgebildet ist, um das Verfahren nach einem der Ansprüche 1 bis 9 zu berechnen und/oder um die Operationen der Datenanalyseeinheit der Vorrichtung nach einem der Ansprüche 9 bis 12 zu implementieren.

## Revendications

1. Procédé d'analyse d'un tissu corporel cible d'un sujet, le procédé comprenant les étapes suivantes :
a) recevoir, dans une unité d'analyse de données, des données d'image du tissu corporel cible et d'un tissu corporel d'arrière-plan adjacent au tissu corporel cible, les données d'image étant obtenues au cours d'une période de temps après l'administration au sujet d'un agent de contraste ;
b) déterminer l'intensité de la lumière émise par une première zone du tissu corporel cible et l'intensité de la lumière émise par une première zone du tissu corporel d'arrière-plan à plusieurs moments de la période de temps, l'intensité de la lumière émise par le tissu corporel cible et le tissu corporel d'arrière-plan à chacun de la pluralité de moments dépendant de la quantité ou de la luminosité de l'agent de contraste présent dans lesdits tissus à chaque moment ;
c) comparer l'intensité de la lumière émise par la première zone du tissu corporel cible et l'intensité de la lumière émise par la première zone du tissu corporel d'arrière-plan au cours de la pluralité de moments ;
d) déterminer, pour une première partie de la période et une seconde partie de la période, la première partie de la période étant antérieure à la seconde partie de la période, la première partie de la période étant une phase d'absorption initiale de l'agent de contraste et la seconde partie de la période étant une phase d'élimination de l'agent de contraste, si :
1) l'intensité de la lumière émise par le tissu corporel cible est inférieure à l'intensité de la lumière émise par le tissu corporel d'arrière-plan pendant la première partie de la période ; et
2) l'intensité de la lumière émise par le tissu corporel cible est supérieure à l'intensité de la lumière émise par le tissu corporel d'arrière-plan pendant la seconde partie de la période ;
où une détermination négative par rapport à 1) et/ou 2) indique que le tissu corporel cible est bénin et une détermination positive par rapport à 1) et/ou 2) indique que le tissu corporel cible est malin ; et
où l'étape d) 2) implique de déterminer si l'intensité de la lumière émise par le tissu corporel cible et l'intensité de la lumière émise par le tissu corporel d'arrière-plan présentent une relation convergente ou divergente au cours de la seconde partie de la période, une relation convergente indiquant que le tissu corporel cible est bénin et une relation divergente indiquant que le tissu corporel cible est malin.

2. Procédé selon la revendication 1, dans lequel le tissu corporel cible est une lésion rectale.

3. Procédé selon la revendication 1 ou la revendication 2, dans lequel les données d'image ont été obtenues tandis que le tissu corporel cible et le tissu corporel d'arrière-plan étaient irradiés par une lumière dans le proche infrarouge.

4. Procédé selon l'une quelconque des revendications précédentes, dans lequel la période de temps dure au moins 5 minutes.

5. Procédé selon l'une quelconque des revendications précédentes, dans lequel l'agent de contraste est le vert d'indocyanine.

6. Procédé selon l'une quelconque des revendications précédentes, dans lequel les données d'image sont reçues au format RVB et l'étape b) comprend de combiner les données des canaux rouge, vert et bleu pour chaque pixel de l'enregistrement vidéo dans la première zone du tissu corporel cible et dans la première zone du tissu corporel d'arrière-plan pour fournir un format en niveaux de gris comprenant un seul point de données pour chaque pixel.

7. Procédé selon l'une quelconque des revendications précédentes, dans lequel la pluralité de points temporels sont séparés par des intervalles allant jusqu'à 120 secondes.

8. Procédé selon l'une quelconque des revendications précédentes, dans lequel la première partie de la période de temps commence à 0 minute dans la période de temps et se termine à un maximum de 2 minutes dans la période de temps.

9. Procédé selon l'une quelconque des revendications précédentes, dans lequel la deuxième partie de la période de temps commence à un maximum de 25 minutes après le début de la période de temps et se termine au moins 30 minutes après le début de la période de temps.

10. Dispositif d'analyse d'un tissu corporel cible d'un sujet, le dispositif comprenant une unité d'inspection visuelle et une unité d'analyse de données ;
dans lequel l'unité d'inspection visuelle comprend une source de lumière visible et une unité de capture de données d'image ; et
dans lequel l'unité d'analyse des données est adaptée pour :
a) recevoir, depuis l'unité de capture de données d'image, des données d'image dudit tissu corporel cible et dudit tissu corporel d'arrière-plan adjacent audit tissu corporel cible, lesdites données d'image étant prises au cours d'une période de temps ;
b) déterminer l'intensité de la lumière émise par une première zone dudit tissu corporel cible et l'intensité de la lumière émise par une première zone dudit tissu corporel d'arrière-plan à une pluralité de moments de ladite période ;
c) comparer ladite intensité de lumière émise par ladite première zone dudit tissu corporel cible et ladite intensité de lumière émise par ladite première zone dudit tissu corporel d'arrière-plan sur ladite pluralité de points temporels ; et
d) déterminer si :
1) ladite intensité de lumière émise par ledit tissu corporel cible est inférieure à ladite intensité de lumière émise par ledit tissu corporel d'arrière-plan pendant une première partie de ladite période de temps ; et
2) ladite intensité de lumière émise par ledit tissu corporel cible est supérieure à ladite intensité de lumière émise par ledit tissu corporel d'arrière-plan pendant ladite deuxième partie de ladite période de temps ;
dans lequel d) 2) comprend de déterminer si l'intensité de la lumière émise par le tissu corporel cible et l'intensité de la lumière émise par le tissu corporel d'arrière-plan ont une relation convergente ou divergente au cours de la seconde partie de la période de temps.

11. Dispositif selon la revendication 10, dans lequel l'unité d'inspection visuelle est un endoscope.

12. Dispositif selon la revendication 10 ou la revendication 11, dans lequel l'unité d'inspection visuelle comprend une source de lumière dans le proche infrarouge.

13. Support lisible par ordinateur comprenant un code configuré pour mettre en œuvre le procédé selon l'une quelconque des revendications 1 à 9 et/ou pour mettre en œuvre les opérations de l'analyse des données du dispositif selon l'une quelconque des revendications 9 à 12.

14. Programme informatique configuré, lorsqu'il est exécuté dans un ordinateur, pour mettre en œuvre le procédé selon l'une quelconque des revendications 1 à 9 et/ou pour mettre en œuvre les opérations de l'unité d'analyse de données du dispositif selon l'une quelconque des revendications 9 à 12.
